# EUROPÄISCHE PATENTANMELDUNG

(11) **EP 0 829 499 A1**
(43) Veröffentlichungstag der Anmeldung: **18.03.1998**
(21) Anmeldenummer: 97112256.9
(22) Anmeldetag: 17.07.1997
(51) Int. Cl.: C08G 18/79, C08G 18/78, C07D 229/00, C09D 175/04, C09D 5/03

(54) **Uretdiongruppenhaltige Polyisocyanate, ein Verfahren zu ihrer Herstellung sowie deren Verwendung**

(30) Priorität: 13.09.1996 DE 19637375
(71) Anmelder: HÜLS AKTIENGESELLSCHAFT, 45764 Marl (DE)
(72) Erfinder: Gras, Rainer, Dr., 44879 Bochum (DE)

(57) **Zusammenfassung**

Harnstoff- und uretdiongruppenhaltige Polyisocyanate folgender Zusammensetzung: 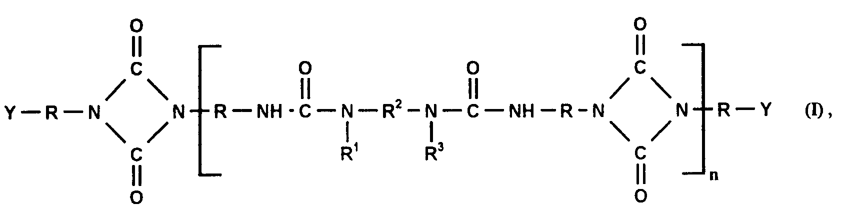 wobei
- Y: = -NCO , 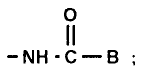
- B: = 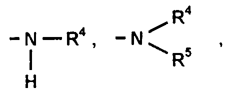 -OR⁴ , 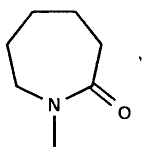 R⁴=N-O- , 1.2.4-Triazol ;
- R: = 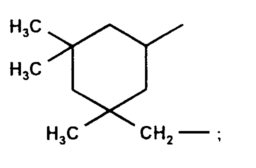
- n: = 1 - 20 ;
- R¹, R³: = höchstens ein linearer oder verzweigter (Cyclo)-Alkylrest mit 1 - 14 C-Atomen oder H und mindestens eine der Formel 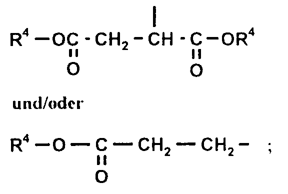
- R²: = R, linearer oder verzweigter (Cyclo)-Alkylrest mit 6 C-Atomen;
- R⁴, R⁵: = gleiche oder verschiedene Kohlenwasserstoffreste mit 1 - 14 C-Atomen
bedeuten.

## Beschreibung

Die Erfindung betrifft uretdiongruppenhaltige Polyisocyanate mit freien, partiell oder total blockierten Isocyanatgruppen, ein Verfahren zur Herstellung sowie deren Verwendung zur Herstellung von Polyurethan(PUR)-Lacksystemen, insbesondere von PUR-Pulverlacken und die danach hergestellten Beschichtungen mit reduziertem Glanz.

Pulverlackbeschichtungen mit reduziertem Glanz finden in den letzten Jahren immer größeres Interesse. Die Ursache dafür ist überwiegend praktischer Art. Glänzende Flächen erfordern ein weitaus höheres Maß an Reinigung als matte Flächen. Darüber hinaus kann es aus sicherheitstechnischen Gründen erforderlich sein, stark reflektierende Oberflächen zu vermeiden.

Die einfachste Lösung, eine Oberfläche mit reduziertem Glanz zu erhalten, besteht darin, den Pulverlackformulierungen je nach gewünschtem Glanzgrad - Matteffekt - kleinere oder größere Mengen an Füllstoff wie Kreide, Bariumsulfat oder fein verteiltes Siliciumdioxid zuzusetzen. Diese Zuschlags- bzw. Hilfsstoffe führen jedoch zur Reduzierung der lacktechnischen Filmeigenschaften wie Flexibilität, Erichsen Tiefung, Kugelschlagfestigkeit, Haftung und Chemikalienfestigkeit.

Die Zugabe von Stoffen, die mit dem Lack unverträglich sind, wie z. B. Wachse oder Cellulosederivate, bewirkt zwar ebenfalls eine Mattierung, aber geringfügige Änderungen während des Extrudierens führen zu Schwankungen im Oberflächenglanz. Die Reproduzierbarkeit des Matteffektes ist nicht gewährleistet.

Überraschenderweise konnten PUR-Pulverlacke mit reduziertem Glanz gefunden werden, die nicht mit den Nachteilen des Standes der Technik behaftet sind, wenn als Vernetzer Umsetzungsprodukte aus Isophorondiisocyanat-Uretdion (abgekürzt IPDI-UD) und disekundären Diaminen eingesetzt werden.

Gegenstand der vorliegenden Erfindung sind somit harnstoff- und uretdiongruppenhaltige Polyisocyanate mit freien, partiell oder total blockierten Isocyanatgruppen folgender Zusammensetzung: 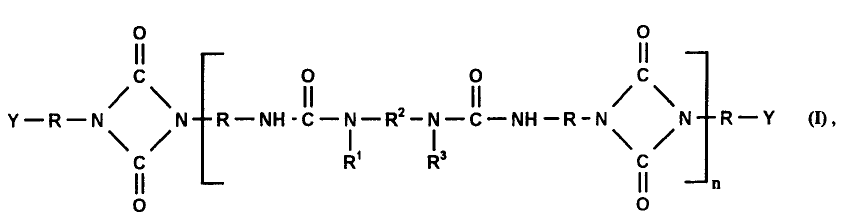 wobei
- Y: = -NCO , 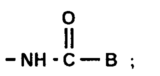
- B: = 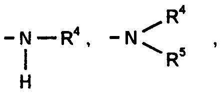 -OR⁴ , 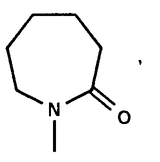 R⁴=N-O- , 1.2.4-Triazol ;
- R: = 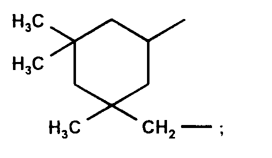
- n: = 1 - 20 ;
- R¹, R³: = höchstens ein linearer oder verzweigter (Cyclo)-Alkylrest mit 1 - 14 C-Atomen oder H und mindestens ein der Formel 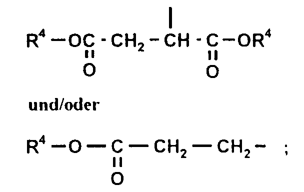
- R²: = R, linearer oder verzweigter (Cyclo)-Alkylrest mit 6 C-Atomen;
- R⁴, R⁵: = gleiche oder verschiedene Kohlenwasserstoffreste mit 1 - 14 C-Atomen
bedeuten.

Die erfindungsgemäßen Polyisocyanate zeichnen sich durch
a) einen Gehalt an freien NCO-Gruppen von ≤ 5, vorzugsweise ≤ 4, insbesondere ≤ 2 Gew.-%,
b) einen Gesamt-NCO-Gehalt (frei und latent und/oder blockiert) von 8 bis 16, vorzugsweise von 9 bis 15, insbesondere von 10 - 14 Gew.-% sowie
c) einen in weiten Grenzen variierenden Schmelzbereich von 70 bis 180 °C
aus.

Sie eignen sich in hervorragender Weise zur Herstellung von PUR-Pulverlackbeschichtungen mit reduziertem Glanz.

Ein weiterer Gegenstand der vorliegenden Erfindung ist ein Verfahren zur Herstellung der erfindungsgemäßen harnstoff- und uretdiongruppenhaltigen Polyisocyanate mit freien, partiell oder total blockierten Isocyanatgruppen gemäß nachstehender Reaktionsgleichung:

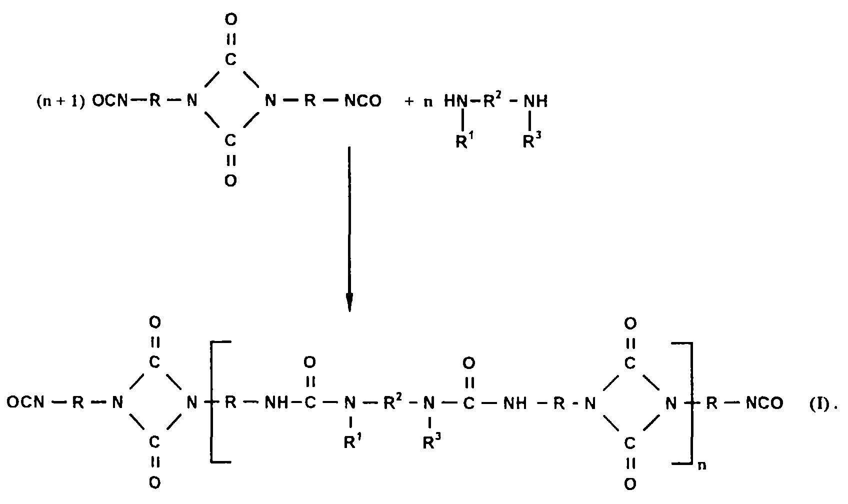

Die freien NCO-Gruppen von (I) können gegebenenfalls partiell oder total mit dem Blockierungsmittel (B) umgesetzt werden.

Bei dem im erfindungsgemäßen Verfahren einzusetzenden Uretdion handelt es sich um das IPDI-Uretdion, wie es in den DE-OSS 30 30 513 und 37 39 549 beschrieben wird, mit einem freien NCO-Gehalt von 16,8 - 18,5 %, d. h., daß mehr oder minder hohe Anteile an Polyuretdion des IPDI im Reaktionsprodukt vorliegen müssen. Der Monomergehalt liegt bei ≤ 1 %. Der Gesamt-NCO-Gehalt des IPDI-Uretdions nach dem Erhitzen auf 180 - 200 °C (0,5 h) beträgt 37,4 - 37,8 %.

Die für das erfindungsgemäße Verfahren eingesetzten sekundären Diamine sind seit längerem bekannt und werden durch Umsetzung von primären Diaminen H₂N-R²-NH₂ mit Malein- oder Fumarsäureester erhalten, wobei pro Mol Malein- bzw. Fumarsäureester 0,5 Mol Diamin in bekannter Weise zur Reaktion kommen. Bei den Diaminen H₂N-R²-NH₂ handelt es sich um aliphatische bzw. (cyclo)aliphatische Diamine wie 1.2-Diaminoethan, 1.3-Diaminopropan, 1.4-Diaminobutan, 1.5-Diaminopentan, 1.6-Diaminohexan, 2-Methyl-1.5-Diaminopentan (DA 51), 2.2.4(2.4.4)-Trimethyl-1.6-diaminohexan (TMD), 2.4'- und/oder 4.4'-Diaminodicyclohexylmethan (HMDA), 1.11-Diaminoundecan, 1.12-Diaminododecan, 1.4- bzw. 1.2-Diaminocyclohexan, m-Hexahydroxylylendiamin (HXDA), 1-Amino-3.5.5-trimethyl-5-aminomethylcyclohexan (Isophorondiamin, abgekürzt IPD).

Aus der Vielzahl der bekannten Malein- bzw. Fumarsäureester wurden in der Hauptsache der Maleinsäurediethyl-, -dibutyl- und -di-2-ethylhexylester verwendet.

Eine vorteilhafte Variante für die Herstellung der erfindungsgemäßen Polyisocyanate stellen disekundäre Diamine dar, die durch Umsetzung einer primären Aminogruppe mit einem o. g. Malen- bzw. Fumarsäureester erhalten werden, d. h. pro Mol primäres Diamin wird ein Mol Maleinsäureester zur Reaktion gebracht und anschließend die zweite Aminogruppe mit einem Acrylsäureester wie Acrylsäuremethyl-, Acrylsäureethyl-, Acrylsäurebutyl-, Acrylsäure-t.-butyl-, Acrylsäure-2-ethylhexylester sekundarisiert. Die Umsetzung erfolgt zwischen 60 und 80 ^{o}C. Selbstverständlich kann die Umsetzung der Aminogruppen mit Malein- bzw. Fumarsäureester und Acrylsäureester auch in umgekehrter Reihenfolge erfolgen.

Eine weitere Variante zur Herstellung der erfindungsgemäßen Polyisocyanate stellt die Umsetzung von Diaminen, welche eine sekundäre und eine primäre Aminogruppe enthalten, mit Malein- bzw. Fumarsäureester oder Acrylsäureester dar, wobei die primäre Aminogruppe sekundarisiert wird.

Manchmal hat es sich als zweckmäßig erwiesen, die freien NCO-Gruppen der uretdiongruppenhaltigen Polyadditionsprodukte zu blockieren. Für den Fall, daß die NCO-Gruppen irreversibel blockiert werden, d. h. beim Einbrennvorgang wird das Blockierungsmittel nicht abgespalten, werden die freien NCO-Gruppen des Uretdion-Diamin-Adduktes mit Monoalkoholen, wie z. B. Methanol, Ethanol, Butanol, 2-Ethylhexanol (EHL), oder sekundären Monoaminen, wie z. B. Dibutylamin (DBA), Di-2-ethylhexylamin, Methylcyclohexylamin, umgesetzt. Für die reversible Blockierung der freien NCO-Gruppen kommen Ketoxime, wie z. B. Acetonoxim, Methylethylketoxim, Acetophenonoxim und Cyclohexanonoxim, Lactame wie Caprolactam sowie Triazole wie 1.2.4-Triazol zum Einsatz.

Die erfindungsgemäßen uretdiongruppenhaltigen Polyadditionsverbindungen werden nach dem nunmehr erläuterten Verfahren hergestellt. Die Herstellung erfolgt so, daß n+1 mol IPDI-Uretdion mit n mol disekundärem Diamin zur Reaktion gebracht werden und dabei die Temperatur nicht über 40 °C steigt. Erfindungsgemäß können die freien NCO-Gruppen vor oder nach der Reaktion mit den disekundären Diaminen mit Blockierungsmitteln zur Reaktion gebracht werden.

Die Umsetzung kann jeweils mit oder ohne Lösemittel erfolgen. Im Falle des Einsatzes eines Lösemittels kann dies ausgewählt werden aus der Gruppe aromatischer Kohlenwasserstoffe, Ester oder Ketone, wie z. B. Toluol, Ethyl- oder Butylacetat, Aceton, Methylethylketon, Methylisobutylketon sowie beliebige Gemische dieser Lösemittel. Bevorzugtes Lösemittel ist Aceton.

In einer bevorzugten Ausführungsform erfolgt die Herstellung der erfindungsgemäßen Verbindungen so, daß zu der acetonischen Lösung des IPDI-Uretdions bei Raumtemperatur das disekundäre Diamin so zudosiert wird, daß die Temperatur der Reaktionslösung nicht über 40 °C steigt. Nach Beendigung der Diaminzugabe ist die Reaktion beendet. Es wird das Aceton abdestilliert, das ist dann der Fall, wenn das Reaktionsprodukt noch freie NCO-Gruppen enthalten soll. Soll jedoch das Reaktionsprodukt keine freien NCO-Gruppen mehr enthalten, schließt sich die Umsetzung mit dem Blockierungsmittel bei ca. 60 °C an. Wenn als Blockierungsmittel Alkohole eingesetzt werden, hat es sich als zweckmäßig erwiesen, IPDI-Uretdion bei ca. 70 °C mit Alkoholen lösemittelfrei umzusetzen und nach erfolgter OH/NCO-Reaktion bei Raumtemperatur die Umsetzung mit dem disekundären Diamin in Aceton durchzuführen.

Wird auf Lösemittel verzichtet, findet die Umsetzung kontinuierlich in einem Intensivkneter statt, wobei bevorzugt ein Zweiwellenextruder eingesetzt wird.

Oft erscheint es zweckmäßig, den Reaktionsablauf zu beschleunigen, insbesondere zur Herstellung der erfindungsgemäßen partiell oder total blockierten Polyisocyanate. Als Katalysatoren kommen organische Zinnverbindungen wie Sn(II)-octoat, Dibutylzinndilaurat (DBTL), Zinnmaleat usw. in Frage, die mit 0,01 bis 0,5 Gew.-%, vorzugsweise 0,03 bis 0,15 Gew.-% zugesetzt werden.

Ein weiterer Gegenstand der vorliegenden Erfindung ist die Verwendung der harnstoff- und uretdiongruppenhaltigen Polyisocyanate zur Herstellung von Polyurethan-Lacksystemen, insbesondere in Kombination mit hydroxylgruppenhaltigen Polymeren und/oder den in der PUR-Chemie üblichen Zuschlagsstoffen zur Herstellung von transparenten und pigmentierten PUR-Pulverlacken. Als Reaktionspartner für PUR-Pulverlacke kommen Verbindungen in Frage, welche solche funktionellen Gruppen tragen, die sich mit Isocyanatgruppen während des Härtungsprozesses in Abhängigkeit von der Temperatur und Zeit umsetzen, z. B. Hydroxyl-, Carboxyl-, Mercapto-, Amino-, Urethan-, (Thio)Harnstoffgruppen. Als Polymere können Polymerisate, Polykondensate und Polyadditionsverbindungen eingesetzt werden.

Grundsätzlich kann jedes Polymer verwendet werden, welches mehr als zwei OH-Gruppen enthält und bei mindestens 70 °C schmilzt. Es sind dies Polyetherpolyole, Polyesteramidpolyole, Polyurethanpolyole, hydroxylierte Acrylatharze usw., deren OH-Gruppen für die Vernetzung mit den erfindungsgemäßen uretdiongruppenhaltigen Polyisocyanaten bestimmt sind. Besonders bevorzugt sind unter den zahlreichen Möglichkeiten für Hydroxylgruppen tragende Polymere im Rahmen der Erfindung Polyesterpolyole. Die besonders bevorzugt eingesetzten hydroxylgruppenhaltigen Polyester haben eine OH-Funktionalität von > 2, eine OH-Zahl von 20 bis 200 mg KOH/g, vorzugsweise 30 bis 150 mg KOH/g, eine Viskosität von < 60 000 mPa·s, vorzugsweise < 40 000 mPa·s, bei 160 °C und einen Schmelzpunkt von > 70 bis ≤ 120 °C, vorzugsweise 75 bis 100 °C.

Solche Polyester können auf an sich bekannte Weise durch Kondensation in einer Inertgasatmosphäre bei Temperaturen von 100 bis 260 °C, vorzugsweise 130 bis 220 ^{o}C, in der Schmelze oder in azeotroper Fahrweise gewonnen werden, wie es in Methoden der Organischen Chemie (Houben-Weyl), Bd. 14/2, 1 - 5, 21 - 23, 40 - 44, Georg Thieme Verlag, Stuttgart, 1963 oder bei C. R. Martens, Alkyd Resins 51 - 59, Reinhold Plastics Appl. Series, Reinhold Publishing Comp., New York und in den DE-OSS 19 57 483, 25 42 191, 30 04 876 und 31 43 060 beschrieben wird.

Das Mischungsverhältnis der hydroxylgruppenhaltigen Polymeren und den erfindungsgemäßen Polyisocyanaten wird in der Regel so gewählt, daß auf eine OH-Gruppe 0,5 - 1,2, bevorzugt 0,8 - 1,1, ganz bevorzugt 1,0, NCO-Gruppe kommt.

Um die Geliergeschwindigkeit der hitzehärtbaren Pulverlacke zu erhöhen, kann man Katalysatoren zusetzen Als Katalysatoren verwendet man Organozinnverbindungen wie Dibutylzinndilaurat (DBTL), Sn(II)-octoat, Dibutylzinnmaleat usw. Die Menge an zugesetztem Katalysator beträgt 0,1 - 5 Gew.-T. auf 100 Gew.-T. des Hydroxylgruppen tragenden Polyesters.

Die Isocyanatkomponente wird für die Herstellung von PUR-Pulverlacken mit dem geeigneten hydroxylgruppenhaltigen Polymeren und gegebenenfalls Katalysatoren sowie Pigmenten und üblichen Hilfsmitteln wie Füllstoffen und Verlaufsmitteln, z. B. Siliconöl, Acrylatharzen, gemischt und in der Schmelze homogenisiert. Dies kann in geeigneten Aggregaten, wie z. B. beheizbaren Knetern, vorzugsweise jedoch durch Extrudieren, erfolgen, wobei Temperaturobergrenzen von 130 bis 140 °C nicht überschritten werden sollen. Die extrudierte Masse wird nach Abkühlen auf Raumtemperatur und nach geeigneter Zerkleinerung zum sprühfertigen Pulver vermahlen. Das Auftragen des sprühfertigen Pulvers auf geeignete Substrate kann nach den bekannten Verfahren, wie z. B. durch elektrostatisches Pulversprühen, Wirbelsintern, elektrostatisches Wirbelsintern, erfolgen. Nach dem Pulverauftrag werden die beschichteten Werkstücke zur Aushärtung 30 bis 10 Minuten auf eine Temperatur von 180 bis 220 °C erhitzt.

Nachfolgend wird der Gegenstand der Erfindung anhand von Beispielen näher erläutert. Die verwendeten Abkürzungen sind in der Beschreibung an den entsprechenden Stellen erklärt.

### A Nichterfindungsgemäße Verbindungen

### I. Reaktionsprodukte aus 1 mol Diamin und 2 mol Maleinsäureester

### Allgemeine Herstellungsvorschrift

Zu 2 mol Malein- bzw. Fumarsäureester wird bei 50 - 60 °C 1 mol Diamin so zugetropft, daß die Temperatur des Reaktionsgemisches nicht über 70 °C steigt. Nach Beendigung der Diaminzugabe wird zur Vervollständigung der Reaktion noch ca. 2 h bei 60 °C weitererhitzt. Für die weitere Umsetzung mit dem Polyisocyanat ist eine weitere Behandlung (Destillation) des Reaktionsgemisches nicht erforderlich.

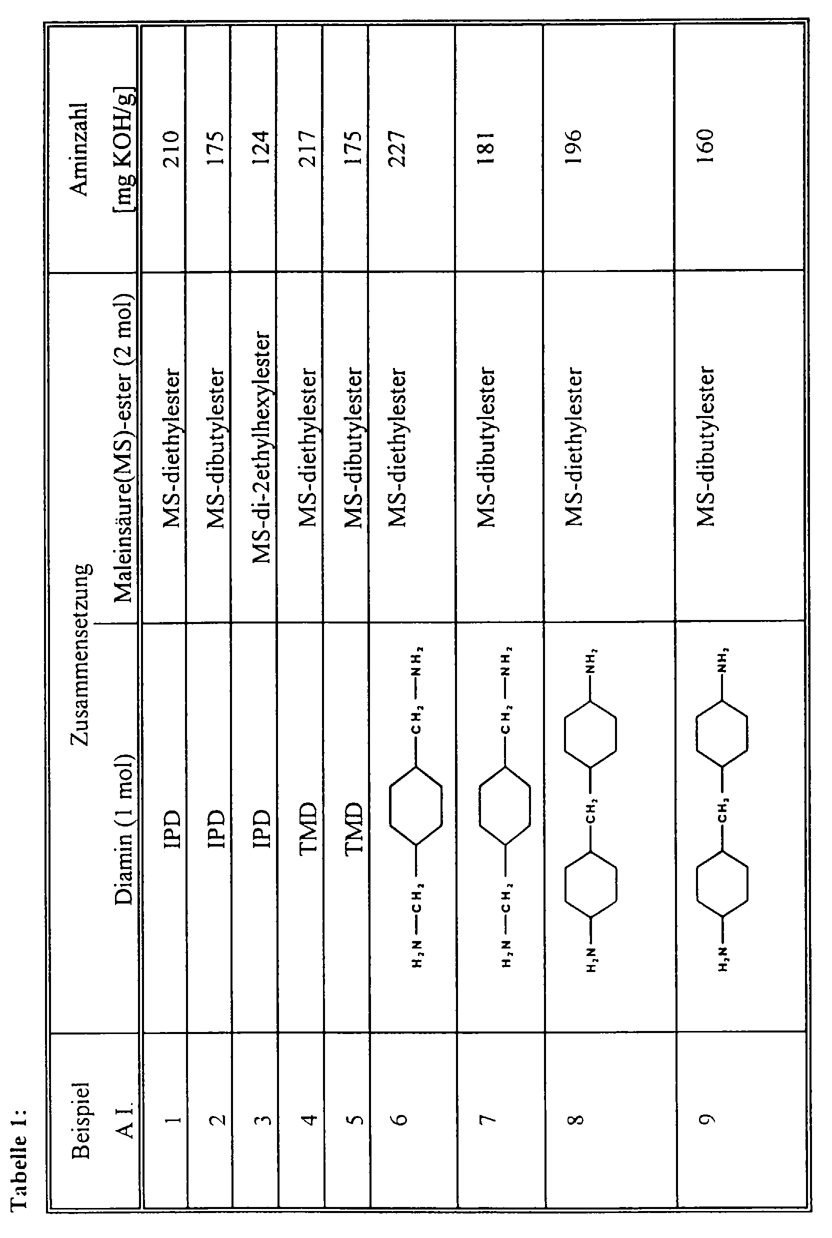

### II. Reaktionsprodukte aus 1 mol Diamin und 1 mol Maleinsäureester und 1 mol Acrylsäureester

### Allgemeine Herstellungsvorschrift

Zu 1 mol Diamin wird bei 60 - 70 °C 1 mol Acrylsäureester so zugetropft, daß die Temperatur des Reaktionsgemisches nicht über 80 °C steigt. Nach Beendigung der Acrylsäureesterzugabe wird so lange bei 70 °C weitererhitzt, bis der Amingehalt den berechneten Wert erreicht hat. Anschließend wird 1 mol Malein- bzw. Fumarsäureester bei 50 - 60 °C so zugetropft, daß die Temperatur der Reaktionsmischung 70 °C nicht übersteigt. Nach Beendigung der Maleinsäureesterzugabe wird zur Vervollständigung der Reaktion noch ca. 2 h bei 70 °C weitererhitzt. Für die weitere Umsetzung mit dem Polyisocyanat ist eine weitere Behandlung (Destillation) des Reaktionsgemisches nicht erforderlich.

**Tabelle 2**

| Beispiel A II. | Zusammensetzung (je 1 mol) | | | Aminzahl [mg KOH/g] |
|---|---|---|---|---|
| | Diamin | MS-ester | Acrylsäure(As)ester | |
| 1 | IPD | -diethylester | As-ethylester | 251 |
| 2 | IPD | -dibutylester | As-methylester | 230 |
| 3 | TMD | -diethylester | As-t.-butylester | 241 |
| 4 | TMD | -dibutylester | As-methylester | 236 |
| 5 | DA 51 | -diethylester | As-methylester | 295 |
| 6 | HMDA | -diethylester | As-ethylester | 231 |
| 7 | HMDA | -dihexylester | As-methylester | 191 |
| 8 | HXDA | -diethylester | As-ethylester | 267 |

### III. Reaktionsprodukte aus 1 mol Diamin und 1 mol Malein- bzw. Fumarsäureester

### Allgemeine Herstellungsvorschrift

Zu 1 mol Diamin wird bei 50 - 60 °C 1 mol Malein- bzw. Fumarsäureester so zugesetzt, daß die Reaktionstemperatur nicht über 70 °C steigt. Nach Beendigung der Esterzugabe wird noch ca. 1 h bei 60 °C weitererhitzt. Nach dem Abkühlen ist das Reaktionsprodukt für die Umsetzung mit Polyisocyanat-Uretdion einsetzbar.

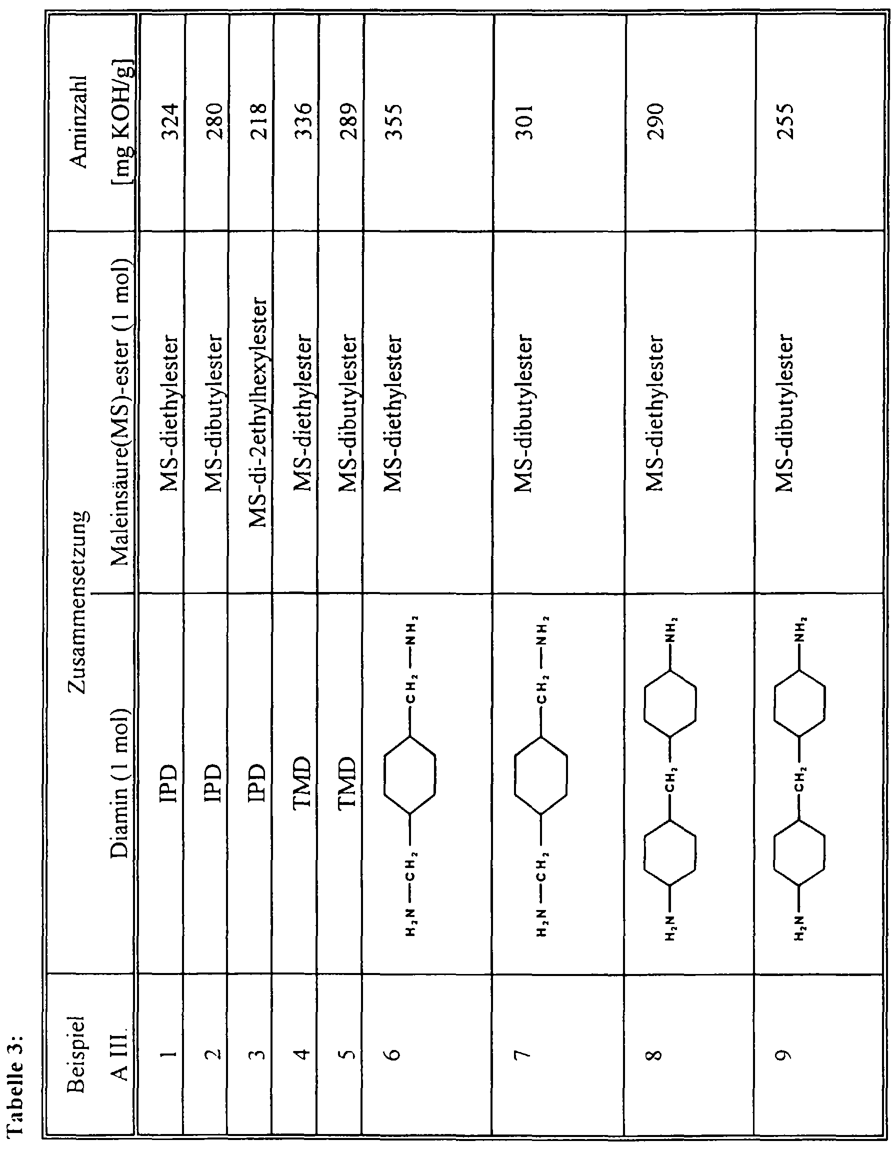

### B Herstellung der erfindungsgemäßen Verbindungen

### Allegmeine Herstellungsvorschrift

Zu der acetonischen Lösung des IPDI-Uretdions wird bei Raumtemperatur das Diamin gemäß Tabelle 1 bis 3 unter intensiver Rührung so zudosiert, daß die Temperatur der Reaktionslösung 40 °C nicht übersteigt. Nach Beendigung der Diaminzugabe ist die Reaktion praktisch beendet, und es erfolgt die Acetonentfernung. Sollen die freien NCO-Gruppen des IPDI-Uretdion/Diamin-Additionsproduktes (Molverhältnis: IPDI-Uretdion: Diamin = (n+1) : n) mit sekundären Monoaminen blockiert werden, so geht man so vor, daß nach der IPDI-Uretdion/Diamin-Reaktion das Blockierungsmittel bei ca. 40 °C portionsweise zudosiert und ca. 1 h bei 60 °C weitererhitzt wird. Danach erfolgt die Acetonentfernung. Sollen die freien NCO-Gruppen des IPDI-Uretdion/Diamin-Additionsproduktes mit Alkoholen (HO-R⁴) blockiert werden, so hat sich folgende Verfahrensweise als vorteilhaft erwiesen:
a) Umsetzung des IPDI-Uretdions mit dem Alkohol in Substanz bei ca. 70 °C (0,5 h),
b) anschließende Abkühlung und Lösung in Aceton,
c) Umsetzung dieser acetonischen Lösung mit den Diaminen.

Das eingesetzte IPDI-Uretdion hatte folgende Kenndaten:
- NCO-Gehalt (frei):: 16,8 - 18,5 Gew.-%
- NCO-Gehalt (gesamt):: 37,4 - 37,8 Gew.-%

Die in den folgenden Tabellen aufgeführten erfindungsgemäßen hamstoff- und uretdiongruppenhaltigen Polyisocyanate wurden entsprechend der allgemeinen Herstellungsvorschrift synthetisiert.

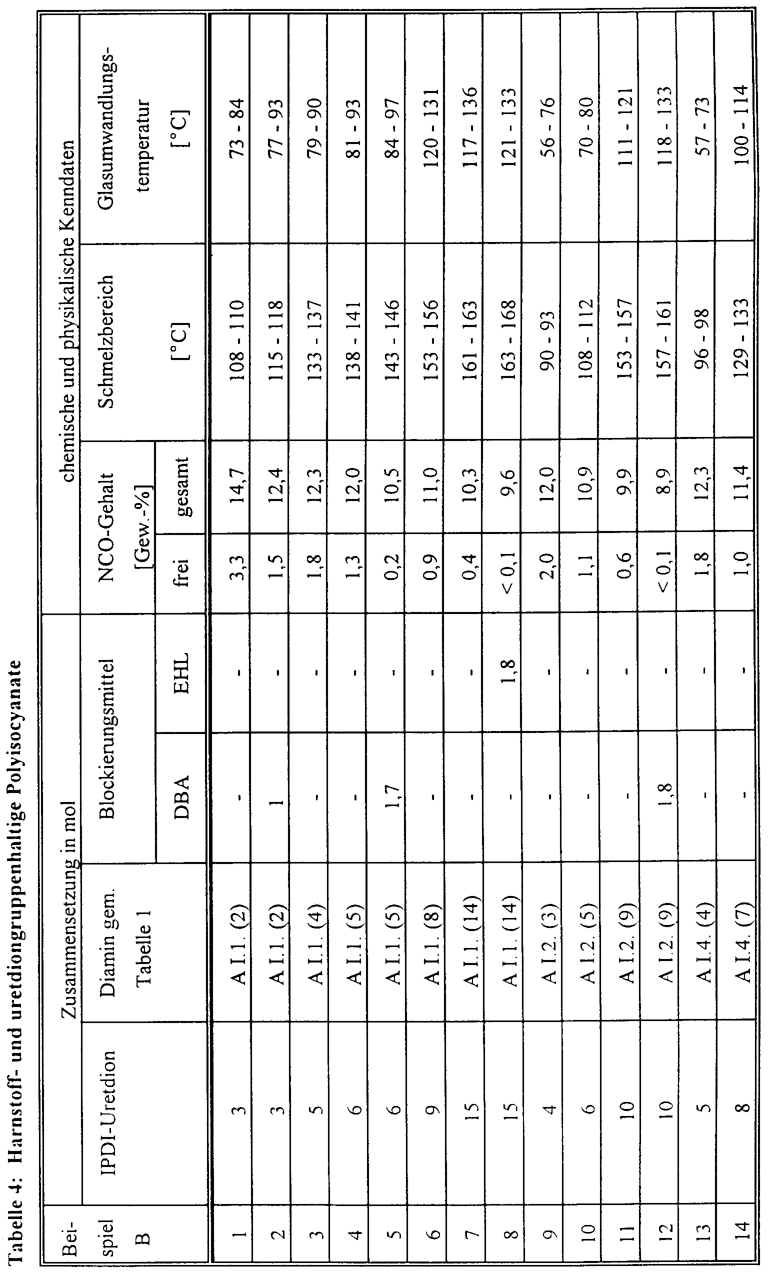

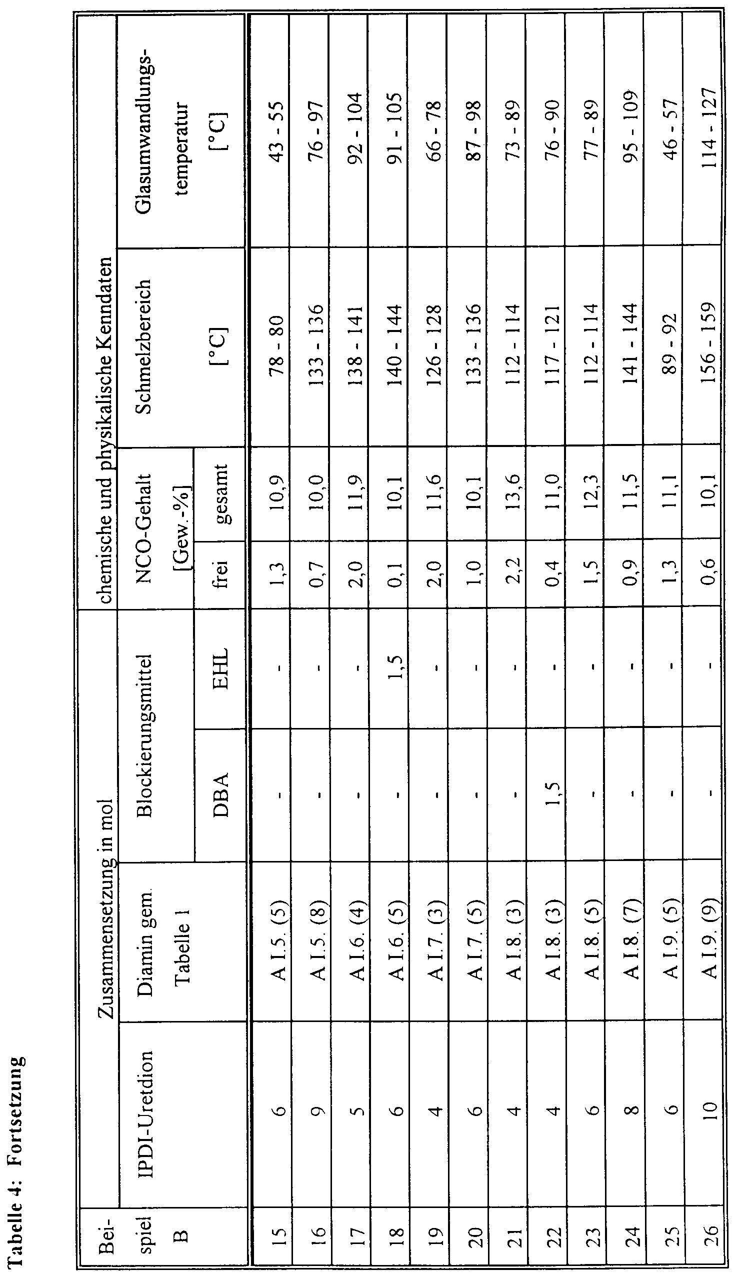

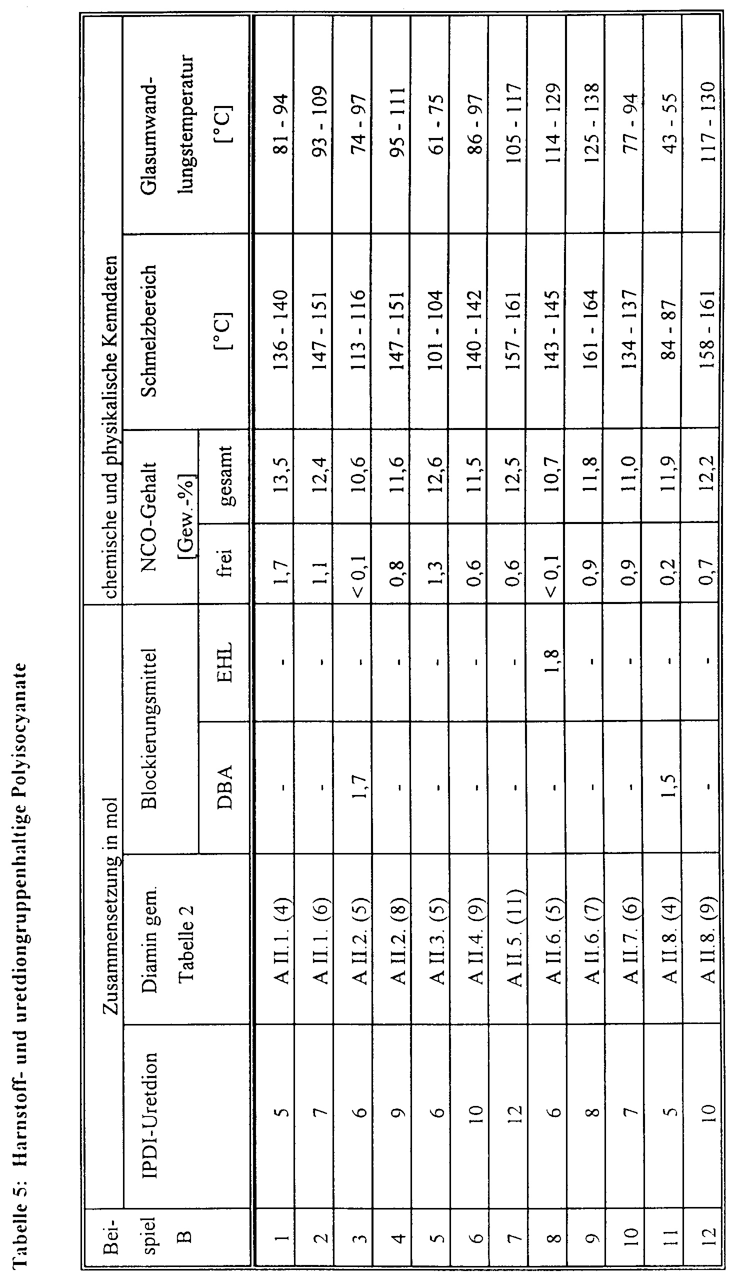

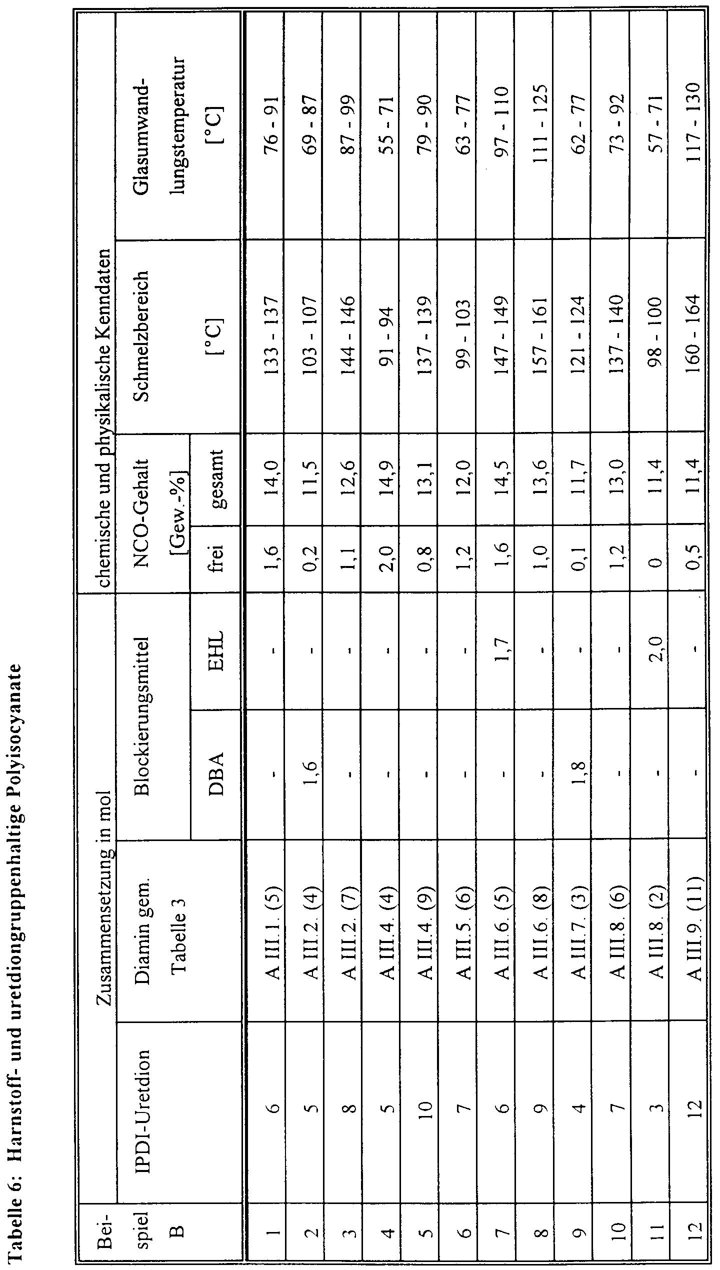

### C Polyolkomponente

### Allgemeine Herstellungsvorschrift

Die Ausgangskomponenten - Terephthalsäure (TS), Dimethylterephthalat (DMT), Hexandiol-1.6 (HD), Neopentylglykol (NPG), 1.4-Dimethylolcyclohexan (DMC) und Trimethylolpropan (TMP) - werden in einen Reaktor gegeben und mit Hilfe eines Ölbades erwärmt. Nachdem die Stoffe zum größten Teil aufgeschmolzen sind, werden bei einer Temperatur von 160 °C 0,5 Gew.-% Di-n-butylzinnoxid als Katalysator zugesetzt. Die erste Methanolabspaltung tritt bei einer Temperatur von ca. 170 °C auf. Innerhalb 6 bis 8 Stunden wird die Temperatur auf 220 bis 230 °C erhöht und innerhalb weiterer 12 bis 15 Stunden die Reaktion zu Ende geführt. Der Polyester wird auf 200 °C abgekühlt und durch Anlegen von Vakuum (1,33 mbar) innerhalb 30 bis 45 Minuten weitgehend von flüchtigen Anteilen befreit. Während der gesamten Reaktionszeit wird das Sumpfprodukt gerührt und ein schwacher N₂-Strom durch das Reaktionsgemisch geleitet.

Tabelle 7 gibt Polyesterzusammensetzungen mit den entsprechenden physikalischen und chemischen Kenndaten wieder.

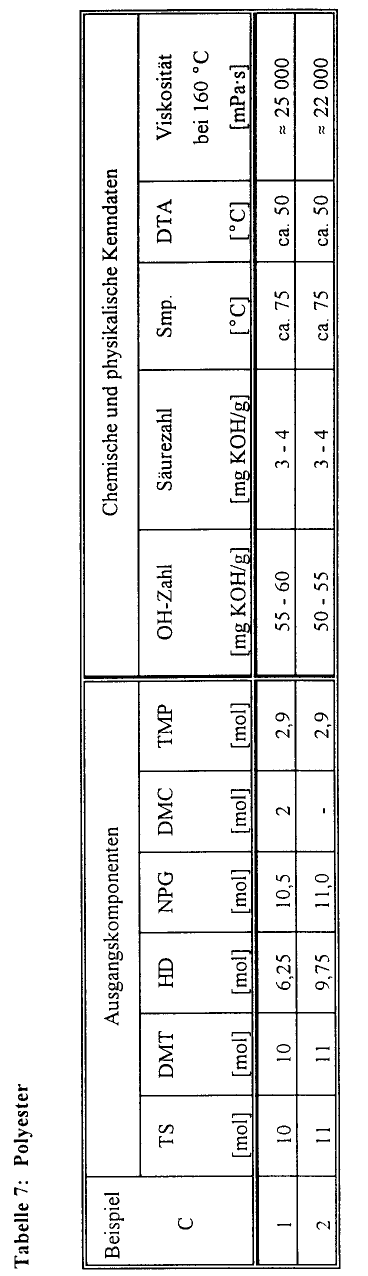

### D Polyurethan-Pulverlacke

### Allgemeine Herstellungsvorschrift

Die zerkleinerten Produkte - harnstoff- und uretdiongruppenhaltige Polyisocyanate (Vernetzer), Polyester, Verlaufsmittel-, ggf Katalysator-Masterbatch - werden ggf. mit dem Weißpigment in einem Kollergang innig vermischt und anschließend im Extruder bis maximal 130 °C homogenisiert. Nach dem Erkalten wird das Extrudat gebrochen und mit einer Stiftsmühle auf eine Korngröße < 100 µm gemahlen. Das so hergestellte Pulver wird mit einer elektrostatischen Pulverspritzanlage bei 60 KV auf entfettete, ggf. vorbehandelte Eisenbleche appliziert und in einem Umlufttrockenschrank bei Temperaturen zwischen 180 und 200 °C eingebrannt.

### Verlaufsmittel-Masterbatch

Es werden 10 Gew.-% des Verlaufsmittels - ein handelsübliches Copolymer von Butylacrylat und 2-Ethylhexylacrylat - in dem entsprechenden Polyester in der Schmelze homogenisiert und nach dem Erstarren zerkleinert.

### Katalysator-Masterbatch

Es werden 5 Gew.-% des Katalysators - DBTL - in dem entsprechenden Polyester in der Schmelze homogenisiert und nach dem Erstarren zerkleinert.

Die Abkürzungen in den folgenden Tabellen bedeuten:
- SD: = Schichtdicke in µm
- ET: = Tiefung nach Erichsen in mm (DIN 53 156)
- GS: = Gitterschnittprüfung (DIN 53 151)
- GG 60 °∢: = Messung des Glanzes nach Gardner (ASTM-D 5233)
- Imp. rev`: = Impact reverse in g·m

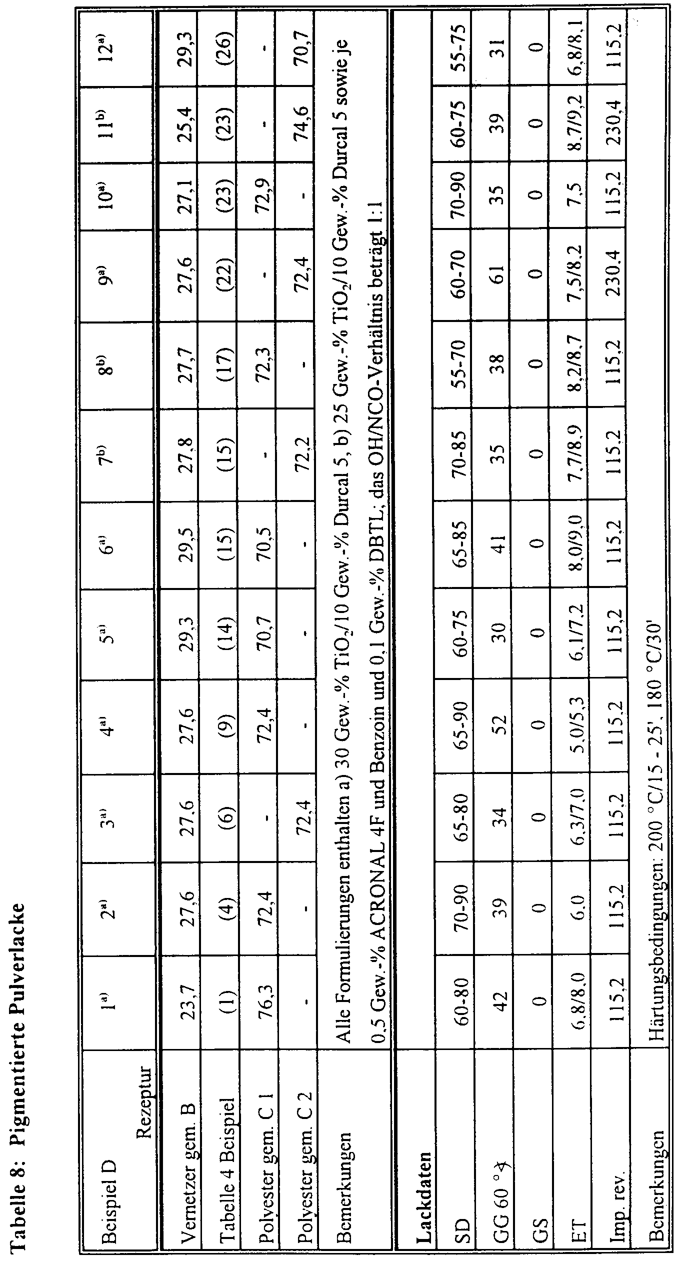

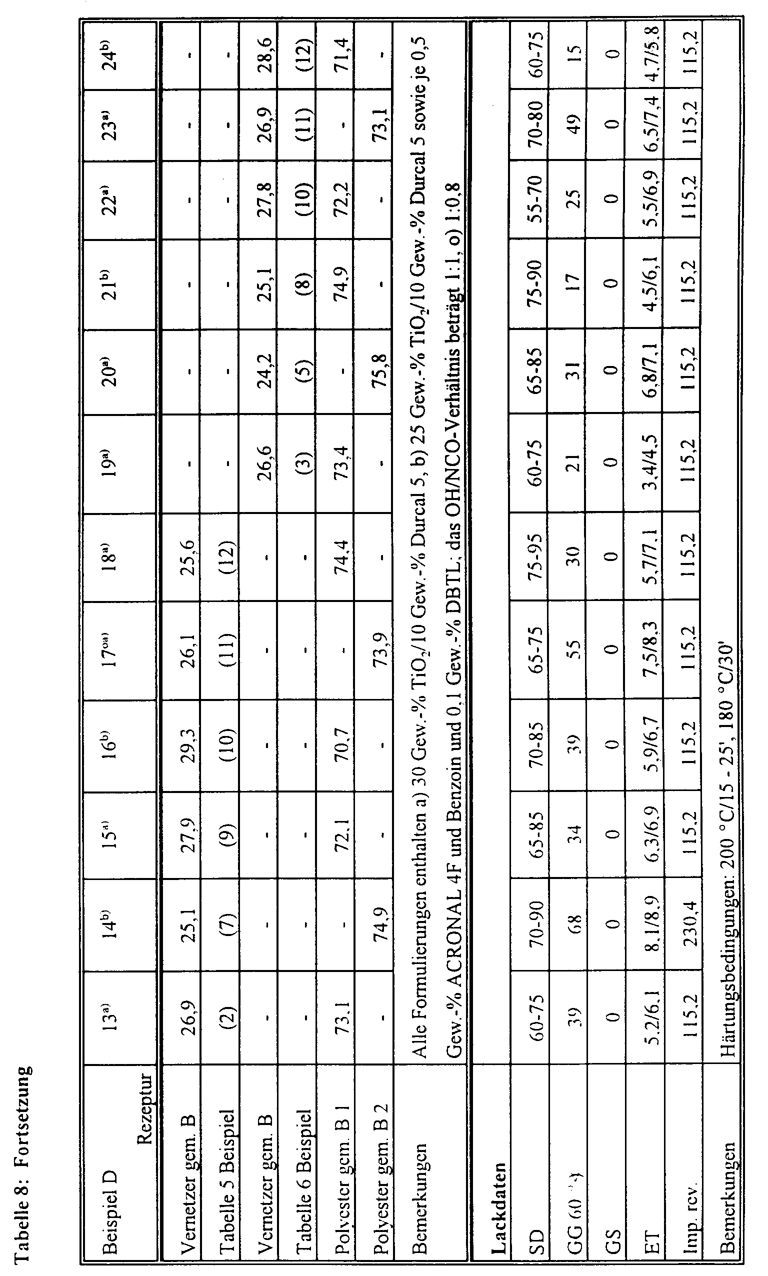

## Patentansprüche

1. Harnstoff- und uretdiongruppenhaltige Polyisocyanate folgender Zusammensetzung: 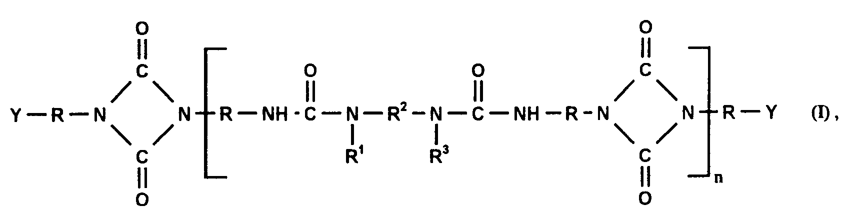 wobei
Y = -NCO , 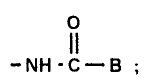
B = 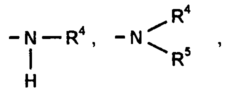 -OR⁴ , 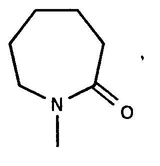 R⁴=N-O- , 1.2.4-Triazol ;
R = 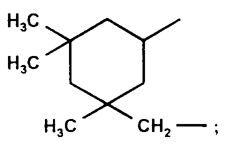
n = 1 - 20 ;
R¹, R³ = höchstens ein linearer oder verzweigter (Cyclo)-Alkylrest mit 1 - 14 C-Atomen oder H und mindestens eine der Formel 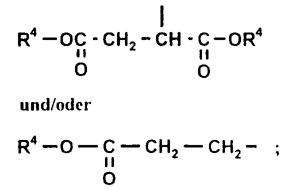
R² = R, linearer oder verzweigter (Cyclo)-Alkylrest mit 6 C-Atomen;
R⁴, R⁵ = gleiche oder verschiedene Kohlenwasserstoffreste mit 1 - 14 C-Atomen
bedeuten.

2. Polyisocyanate nach Anspruch 1,
dadurch gekennzeichnet,
daß sie
a) einen Gehalt an freien NCO-Gruppen von ≤ 5,
b) einen Gesamt-NCO-Gehalt (frei und latent und/oder blockiert) von 8 bis 16 Gew.-%,
sowie
c) einen variierenden Schmelzbereich von 70 bis 180 °C
aufweisen.

3. Polyisocyanate nach den Ansprüchen 1 bis 2,
dadurch gekennzeichnet,
daß sie Reste der Reaktionsprodukte von Malein- und/oder Fumarsäureester und Diamine ausgewählt aus 1.2-Diaminoethan, 1.3-Diaminopropan, 1.4-Diaminobutan, 1.5-Diaminopentan, 1.6-Diaminohexan, 2-Methyl-1.5-Diaminopentan (DA 51), 2.2.4(2.4.4)-Trimethyl-1.6-diaminohexan (TMD), 2.4'- und/oder 4.4'-Diaminodicyclohexylmethan (HMDA), 1.11-Diaminoundecan, 1.12-Diaminododecan, 1.4- bzw. 1.2-Diaminocyclohexan, m-Hexahydroxylylendiamin (HXDA), 1-Amino-3.5.5-trimethyl-5-aminomethylcyclohexan (Isophorondiamin, abgekürzt IPD), enthalten.

4. Polyisocyanate nach Anspruch 3,
dadurch gekennzeichnet,
daß Maleinsäurediethyl-, -dibutyl- und -di-2-ethylhexylester eingesetzt werden.

5. Polyisocyanate nach den Ansprüchen 1 bis 4,
dadurch gekennzeichnet,
daß sie mit Methanol, Ethanol, Butanol, 2-Ethylhexanol (EHL), Dibutylamin (DBA), Di-2-ethylhexylamin, Methylcyclohexylamin, Acetonoxim, Methylethylketoxim, Acetophenonoxim, Cyclohexanonoxim, Caprolactam und 1.2.4-Triazol blockiert sind.

6. Verfahren zur Herstellung der erfindungsgemäßen harnstoff- und uretdiongruppenhaltigen Polyisocyanate mit freien, partiell oder total blockierten Isocyanatgruppen gemäß nachstehender Reaktionsgleichung:

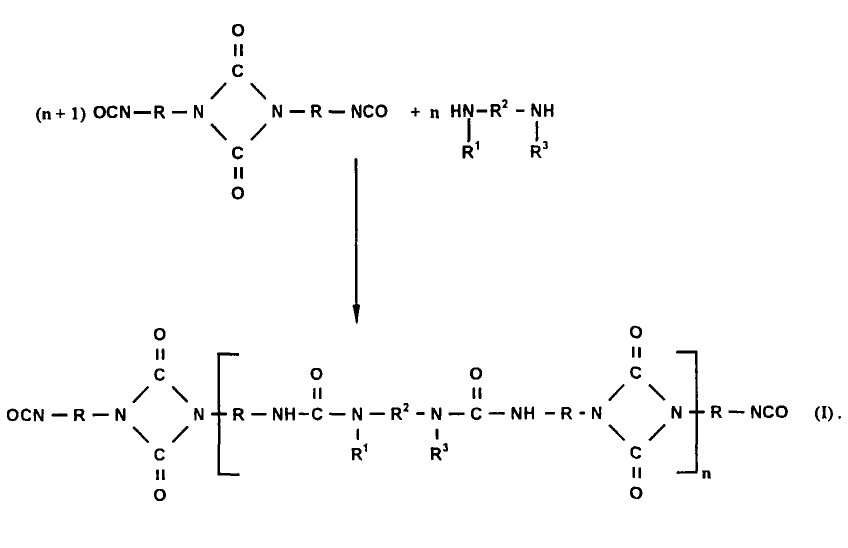

7. Verfahren nach Anspruch 6,
dadurch gekennzeichnet,
daß die Umsetzung in einem Lösemittel erfolgt.

8. Verfahren nach Anspruch 6,
dadurch gekennzeichnet,
daß ohne Lösemittel kontinuierlich in einem Intensivkneter umgesetzt wird.

9. Verfahren nach Anspruch 8,
dadurch gekennzeichnet,
daß in einem Zweiwellenextruder umgesetzt wird.

10. Verwendung der harnstoff- und uretdiongruppenhaltigen Polyisocyanate zur Herstellung von PUR-Lacksystemen, insbesondere von PUR-Pulverlacken und die danach hergestellten Beschichtungen mit reduziertem Glanz.
